# EUROPEAN PATENT APPLICATION

(11) **EP 0 875 562 A1**
(43) Date of publication of application: **04.11.1998**
(21) Application number: 97944195.3
(22) Date of filing: 21.10.1997
(51) Int. Cl.: C12N 9/04, C12N 15/53, C12P 41/00, C12P 7/04

(54) **NOVEL SECONDARY ALCOHOL DEHYDROGENASE, PROCESS FOR PREPARING SAID ENZYME, AND PROCESS FOR PREPARING ALCOHOLS AND KETONES USING SAID ENZYME**

(30) Priority: 22.10.1996 JP 279092/96
(71) Applicant: DAICEL CHEMICAL INDUSTRIES, LTD., Osaka-fu 590 (JP)
(72) Inventor: YAMAMOTO, Hiroaki, Tsukuba-shi, Ibaraki 305 (JP); KAWADA, Naoki, Tsukuba-shi, Ibaraki 305 (JP); MATSUYAMA, Akinobu, Tsukuba-shi, Ibaraki 305 (JP)
(74) Representative: VOSSIUS & PARTNER
(86) International application number: JP9703800
(87) International publication number: WO9817788

(57) **Abstract**

Secondary alcohol dehydrogenase having broad specificity to substrates and high stereoselectivity is provided. By reducing ketones using the enzyme, alcohols, particularly, optically active alcohols are efficiently produced. Ketones are also efficiently produced by oxidizing alcohols using the enzyme.

## Description

### Field of the Invention

The present invention relates to novel secondary alcohol dehydrogenase useful for producing alcohol, aldehyde and ketone, particularly for producing optically active alcohol, a method of producing said enzyme, and a method of producing alcohol, aldehyde, and ketone, particularly producing optically active alcohol by utilizing said enzyme.

### Background of Invention

Known dehydrogenases for secondary alcohol such as phenylethanol produced by microorganisms include secondary alcohol dehydrogenase derived from *Lactobacillus kefir,* which requires nicotinamide adenine dinucleotide phosphate (hereinafter abbreviated as NADP⁺) as a coenzyme (JP-A-Hei 4-330278), and alcohol dehydrogenase derived from *Thermoanaerobium brockii (* J. Am. Chem. Soc. *108:* 162-169, 1986). There are reports about secondary alcohol dehydrogenases requiring NAD⁺ as a coenzyme, including: *Pichia* sp. NRRL-Y-11328 (Eur. J. Biochem. *101:* 401-406, 1979), *Pseudomonas* sp. SPD6 (Bioorg. Chem. *19*: 398-417, 1991), *Pseudomonas fluorescens* NRRL B-1224 (JP-A-Sho 59-17982), *Pseudomonas maltophilia* MB11L (FEMS Microbiol. Lett. *93*: 49-56, 1992), *Pseudomonas* sp. PED (J. Org. Chem. *57*: 1526-1532, 1992), *Pseudomonas* sp. ATCC 21439 (Eur. J. Biochem. *119*: 359-364, 1981), *Candida boidinii* SAHM (Biochem. Biophys. Acta. *716*: 298-307, 1992), *Mycobacterium vaccae* JOB- 5 (J. Gen. Microbiol. *131*: 2901-2907, 1985), *Rhodococcus rhodochrous* PNKb1 (Arch. Microbiol. *153*: 163-168, 1990), *Comamonas terrigena* (Biochem. Biophys. Acta. *661*: 74-86, 1981), *Arthrobacter* sp. SBA (JP-A-Sho 51-57882), and *Candida parasilosis* IFO 1396 (JPA-Hei 7-231789).

However, the stereoselectivity of these secondary alcohol dehydrogenases is not sufficient. For example, secondary alcohol dehydrogenase derived from *Candida parasilosis* IFO 1396 is the only enzyme reported so far to have an activity of generating 2-butanone by streoselective oxidation of (S)-2-butanol from 2-butanol, which are most frequently used as a substrate to secondary alcohol dehydrogenase (Enzymes produced by *Pseudomonas* sp. ATCC 21439, Pseudomonas sp. SPD6, *Comamonas terrigena, Candida boidinii* SAHM and *Pichia* sp. NRRL-Y-11328 preferentially oxidize R forms. The enzyme produced by *Pseudomonas fluorescens* NRRL B-1224 shows no stereoselectivity, and there has been no report about stereoselectivities of the enzymes produced by *Mycobacterium vaccae* JOB-5, *Rhodococcus rhodochrous* PNKb1, *Pseudomonas* sp. PED, and *Pseudomons maltophilia* MB11L). Although it has been reported that S-form of 2-butanol is preferentially oxidized by primary alcohol dehydrogenase (SADH 1) derived from *Saccharomyces cerevisiae* (Arch. Biochem. Biophys. *126*: 933-944, 1968; J. Biol. Chem. *268*: 7792-7798, 1993), its activity is extremely low as approximately 1% relative to ethanol and this enzyme cannot be put into practice. Under these situations, secondary alcohol dehydrogenase possessing high stereoselectivity and broad substrate specificity has been needed to be found.

### Disclosure of Invention

The present inventors paid attention to the fact that *Geotrichum candidum* can act on racemic 1,3-butanediol to stereoselectively oxidize only its S-form, thereby generating 4-hydroxy-2-butane and retaining (R)-1,3-butanediol (JP-B-Hei 6-95951). We purified (S)-1,3-butanediol dehydrogenase produced by *Geotrichum candidum* and examined its enzymochemical properties. As a result, it has been found that this enzyme has such high stereoselectivity as preferentially oxidizing S-form of not only (S)-1,3-butanediol but (S)-phenylethanol, (S)-3-hydroxybutyric acid ester, (S)-2-octanol, and the like, thereby achieving the present invention.

Thus, the present invention relates to an enzyme having the following physicochemical properties:

### (1) Action

The enzyme produces ketone or aldehyde by oxidizing alcohol, in the presence of NAD⁺ (nicotinamide adenine dinucleotide) as a coenzyme. It also produces alcohol by reducing ketone or aldehyde, in the presence NADH (reduced form of nicotinamide adenine dinucleotide) as a coenzyme;

### (2) Substrate specificity

Aliphatic alcohols that may be substituted with an aromatic group are the substrates for the oxidation reaction. The enzyme shows higher activity on secondary alcohols than primary alcohols. It preferentially oxidizes S-form of phenylethanol. Aliphatic aldehydes or ketones that may be substituted with an aromatic group are the substrates for the reduction reaction; and

### (3) Molecular weight

Approximately 51,000, if determined by SDS-PAGE., while approximately 107,000, if determined by gel filtration.

Physicochemical and enzymatic properties of the enzyme of the present invention other than the above are as follows:

### (4) Optimum pH

Optimum pH for the oxidation of (S)-1,3-butanediol ranges from 8.0 to 9.0, and that for the reduction of 4-hydroxy-2-butanone is 7.0;

### (5) pH Range for enzyme stability

The enzyme is relatively stable in the range of pH 9-11;

### (6) Optimal temperature range

The optimum temperature for the oxidation of (S)-1,3-butanediol is 55°C;

### (7) stability

The enzyme is relatively stable up to 30°C;

### (8) Inhibition

The enzyme is perfectly inhibited by p-chloromercuribenzoic acid (PCMB), an SH reagent, or iodoacetamide (IAA). It is also inhibited by heavy metals such as mercury chloride or zinc chloride and by high concentration of ethylenediaminetetraacetic acid or 2-mercaptethanol;

### (9) Purification method

The enzyme can be purified using a suitable combination of methods including fractionation of proteins based on difference in their solubility (e.g., precipitation with organic solvent and salting out by ammonium sulfate or the like), cation exchange chromatography, anion exchange chromatography, gel filtration, hydrophobic chromatography, and affinity chromatography using chelates, pigments, or antibodies. For example, the yeast cells are disrupted and treated by protamine sulfate, ammonium salfate precipitation, anion-exchange chromatography with DEAE-Toyopearl, Blue-Sepharose affinity chromatography, hydrophobic chromatography with Butyl-Toyopearl, gel filtration with TSK G3000SW, and anion-exchange chromatography with Mono Q. Consequently, the enzyme can be purified to the degree that almost one single band of the protein is obtained by polyacrylamide gel electrophoresis.

In the present invention, secondary alcohol dehydrogenase activity was determined by allowing the enzyme to react in the reaction mixture containing potassium phosphate buffer (pH 8.0, 50 µmol), 2.5 µmol NAD⁺, and 50 µmol (S)-1,3-butanediol at 30°C and measuring an increase of absorbance at 340 nm resulting from generation of NADH. One unit was defined as the amount of enzyme catalyzing generation of 1 µmol of NADH per minute.

As for secondary alcohol dehydrogenase produced by micoroorganisms belonging to the genus *Geotrichum,* the method of producing optically active 3-hydroxybutyric acid ester from *Geotrichum candidum* has been known (Helv. Chim. Acta. *66*: 485-488, 1983), but the enzyme involved in this reduction reaction has not been identified. Recently, some reports disclose a method for producing optically active alcohols using acetone powder of *Geotrichum candidum* IFO 4597, implying participation of alcohol dehydrogenase (Tetrahedron Lett. *37*: 1629-1632, 1996; Tetrahedron Lett. *37*: 5727-5730, 1996). However, these reports only describe that NADP⁺ acts more effectively than NAD⁺ as a coenzyme in the reduction reaction, but do not describe enzymochemical properties that would be useful for identification of the enzyme. There is a report showing that an enzyme capable of reducing 4-chloro-3-oxobutyric acid ester to (S)-4-chloro-3-hydroxybutyric acid ester was partially purified from *Geotrichum candidum* SC 5469 (Enzyme Microb. Technol. *14*: 731-738, 1992). Although the purity of that enzyme was too low to specify its characteristics, its molecular weight is 950,000 and it requires NADP as a coenzyme. Thus, this enzyme is distinctly different in such properties from the enzyme of the present invention. Moreover, a description regarding secondary alcohol dehydrogenase derived from *Geotrichum* sp. WF 9101 is found in *"*Biosci. Biotech. Biochem. *60*: 1191-1192, 1996*"*, but it does not reveal a purification method of the enzyme and enzymochemical properties of the purified enzyme such as molecular weight, optimum pH, and optimum temperature.

The present invention also relates to a DNA encoding an enzyme having the following physicochemical properties:

### (1) Action

The enzyme generates ketone or aldehyde by oxidizing alcohol, in the presence of NAD⁺ (nicotinamide adenine dinucleotide) as a coenzyme. It also generates alcohol by reducing ketone or aldehyde, in the presence of NADH (reduced form of nicotinamide adenine dinucleotide) as a coenzyme;

### (2) Substrate specificity

Aliphatic alcohols that may be substituted by an aromatic group are the substrates for the oxidation reaction. The enzyme shows higher activity on secondary alcohols than primary alcohols. S-form of phenylethanol is preferentially oxidized. Aliphatic aldehydes or ketones that may be substituted with an aromatic group are the substrate for the reduction reaction; and

### (3) Molecular weight

Approximately 51,000, if determined by SDS-PAGE, while approximately 107,000, if determined by gel filtration.

By means of activity staining using replicas as described below, one skilled in the art can readily prepare the DNA of the present invention.

Cells of a microorganism belonging to genus *Geotrichum* capable of producing secondary alcohol dehydrogenase are cultured, converted to spheroplast by cell wall degradation enzyme treatment, and a chromosomal DNA is prepared by the standard method (e.g., J. Biol. Chem. *268*: 26212-26219, 1993; Meth. Cell. Biol. *29*: 39-44, 1975). The purified chromosomal DNA is completely or partially digested with appropriate restriction endonuclease (e.g., *Hin*dIII, *Eco*RI, *Bam*HI, *Sau*3AI), and the resulting DNA fragment of about 2-8 kb is introduced into an expression vector for *E. coli* such as pUC18 (Takara Shuzo), pKK223-3 (Pharmacia), pET derivatives (Takara Shuzo etc.), and pMAL-p2 (NEB). The thus-obtained recombinant plasmid was used to transform cells of *E. coli* strain (e.g., JM109) and the tranformants are cultured on the LB medium plate (10 g Bacto-Tryptone, 5 g Bacto-Yeast extract, 10 g NaCl, 15 g/L Bacto-Agar) containing antibiotic appropriate for the plasmid to effect gene expression by adding an appropriate inducer and the like (for example, adding IPTG if the plasmid has a *lac, trp,* or *trc* promoter) or raising the temperature.

Colonies of the transformants thus obtained are transferred from the plates to filter or the like (this is called replica). The cells are lysed on the replica with lysozyme or chloroform (for example, by allowing the cells to stand in a 10 mg/mL solution of lysozyme for about 30 minutes at room temperature). The replica is immersed and reacted in a reaction mixture containing a substrate such as (S)-1,3-butanediol by soaking the replica into reaction solution containing the substrate, NAD⁺ ,phenazine methosulfate (PMS), and nitro blue tetrazolium (NTB) (for example, a reaction mixture containing 100 mM (S)-1,3-butanediol, 1.3 mM NAD⁺, 0.128 mM PMS, and 0.48 mM NBT). As a result, only the tranformants conferred by a plasmid an ability to produce secondary alcohol dehydrogenase derived from genus *Geotrichum* develop violet color. Since *E. coli* strains JM109 and HB101, which are employed as host, and those transformed with plasmid such as pUC18 and pKK223-3 have no (S)-1,3-butanediol dehydrogenase activity, they do not color violet by the above activity staining method using replica.

The DNA region encoding the secondary alcohol dehydrogenase gene can be specified as follows. Namely, a plasmid is prepared from the transformants that have colored and the plasmid is used to prepare plasmids lacking a portion of the insert DNA fragment by digestion with restriction enzyme or endnuclease. Then, *E. coli* cells transformed with the resulting deletion plasmids are examined by the replica method as to whether they have ability to produce secondary alcohol dehydrogenase. The specified DNA region is sequenced to identify an open reading frame based on the initiation codon, the termination codon, the molecular weight of the translated product, and the like information. Thus, the DNA encoding secondary alcohol dehydrogenase produced by the genus *Geotrichum* can be cloned.

The microorganisms having ability to produce secondary alcohol dehydrogenase that are used as genetic sources for the above cloning include any strains belonging to genus *Geotrichum,* mutants and variants thereof and capable of producing secondary alcohol dehydrogenase. It is particularly preferable as such a microorganism to use *Geotrichum candidum* IFO 4601, IFO 5368, and IFO 5767, *Geotrichum capitatum* JCM 3908, *Geotrichum eriense* JCM 3912, *Geotrichum fermentans* IFO 1199 and CBS 2143, *Geotrichum fragrans* JCM 1794, *Geotrichum klebahnii* JCM 2171, and *Geotrichum rectangulatum* JCM 1750.

It was reported that, when a lipase gene derived from the genus *Geotrichum* was cloned, a regulatory region cloned in association with the open reading frame was properly recognized by *E. coli,* the lipase gene was normally expressed to exhibit lipase activity (European Patent No. 243338). It has been also reported that genes derived from the genus *Geotrichum* have no intron (J. Biochem. *113*: 776-780, 1993). In view of these report, it is very likely that the DNA encoding secondary alcohol dehydrogenase produced by the genus *Geotrichum* can be expressed functionally in the cells of *E. coli*. A gene for secondary alcohol dehydrogenase from *Geotrichum* can be expressed in intact form or as a fusion protein if its open reading frame is linked downstream of the promoter under the control of the promoter, using an expression vector for *E. coli* such as pUC18, pKK223-3, pET, and pMAL-p2.

There is a possibility, however, that the secondary alcohol dehydrogenase gene from *Geotrichum* will not be functionally expressed even if the gene is properly positioned downstream of the promoter of *E. coli* that functions in *E. coli* (e.g. intron is included in the gene). In such a case, instead of randomly inserting a chromosomal DNA into a plasmid for *E. coli,* messenger RNA (hereinafter abbreviated as mRNA) is prepared from *Geotrichum,* cDNA is prepared from mRNA by using reverse transcriptase, and the cDNA is introduced into an expression vector fo*r E. coli* or yeast to functionally express the gene. In this occasion, *Saccharomyces cerevisiae* can be used as a yeast host-vector system. Any one of *Saccharomyces cerevisiae* strains AB1380, INVSc2, and BJ2168 does not have (S)-1,3-butanediol dehydrogenase activity and do not color by the activity staining method using replica. In the activity staining method using replica in yeast, the method fo*r E. coli* can be employed except that zymolyase should be used instead of lysozyme, which is an enzyme for cell lysis in *E. coli.*

The enzyme of the present invention or the transformant producing the enzyme or treated products thereof can be used to produce alcohols by acting it on ketones or aldehydes to reduce them. The enzyme of the present invention or the transformant producing the enzyme or treated products thereof can also be used to produce optically active alcohols by acting it on asymmetric ketones to reduce them, utilizing the broad substrate specificity and high level of stereoselectivity of the enzyme of the present invention. For example, it is possible to produce optically active alcohols such as (S)-1,3-butanediol from 4-hydroxy-2-butanone, (S)-phenylethanol from acetophenone, (S)-2-butanol from 2-butanone, (S)-2-octanol from 2-octanone, (S)-3-hydroxy-butyric acid ester from 3-oxobutyric acid ester, and (R)-4-chloro-3-oxobutyric acid ester from 4-chloro-3-oxobutyric acid ester.

Furthermore, the enzyme of the present invention or the transformant producing the enzyme or treated products thereof can be used to produce ketones or aldehydes by acting it on alcohols to oxidize them.

Moreover, the enzyme of the present invention or the transformant producing the enzyme or treated products thereof can he used to produce optically active alcohols by utilizing the ability of secondary alcohol dehydrogenase to asymmetrically oxidize racemic alcohols as a substrate. In other words, optically active alcohols are produced by preferentially oxidizing one form of the optically active alcohols and recovering the remaining optically active alcohol. For example, it is possible to obtain (R)-1,3-butanediol from (RS)-1,3-butanediol, (R)-phenylethanol from (RS)-phenylethanol, (R)-3-hydroxybutyric acid ester from (RS)-3-hydroxybutyric acid ester, and (S)-4-chloro-3-hydroxybutyric acid ester from (RS)-4-chloro-3-hydroxybutyric acid ester.

According to the present invention, the term "enzyme" is not limited to purified enzyme but includes partially purified one. In the present invention, the term "treated products of transformants" refers to products obtained by subjecting a heterologous organism, which has a gene encoding the enzyme of the invention introduced thereinto and is capable of expressing it functionally, to a treatment for modifying permeability of cell walls, such as acetone precipitation, lyophilization, mechanical and enzymatical disruption of cell walls, treatment with a surfactant, treatment in an organic solvent, or the like. The heterologous host includes, for example, microorganisms belonging to genus *Escherichia, Bacillus, Serratia, Pseudomonas, Brevibacterium, Corynebacterium, Streptococcus, Lactobacillus, Saccharomyces, Kluyveromyces, Schizosaccharomyces, Zygosaccharomyces, Yarrowia, Trichosporon, Rhodosporidium, Hansenula, Pichia, Candida, Neurospora, Aspergillus, Cephalosporium,* and *Trichoderma.*

NADH is generated from NAD⁺ concomitantly with the oxidation reaction catalyzed by secondary alcohol dehydrogenase. Regeneration of NAD⁺ from NADH can be effected by using an enzyme (system) contained in microorganisms, which enables regeneration of NAD⁺ from NADH or by adding to the reaction system a microorganism or an enzyme capable of producing NAD⁺ from NADH, for example, glutamate dehydrogenase, NADH oxidase, NADH dehydrogenase, and the like. Utilizing the substrate specificity of the enzyme of the present invention, the substrate for the reduction reaction, such as acetone, may be added to the reaction system to concurrently effect regeneration of NAD⁺ from NADH by the action of the secondary alcohol dehydrogenase by itself.

Further, NAD⁺-reducing ability (e.g., glycolysis) of microorganisms can be utilized to regenerate NADH from NAD⁺ that has been concomitantly generated from NADH in the reduction reaction. Such ability can be reinforced by adding glucose or ethanol to the reaction system. Alternatively, microorganisms capable of generating NADH from NAD⁺, or treated products or enzyme thereof may be added to the reaction system. For example, regeneration of NADH can be carried out using microorganisms containing formate dehydrogenase, glucose dehydrogenase, or malate dehydrogenase, or treated products or enzyme thereof. Utilizing the property of the secondary alcohol dehydrogenase of the present invention, NADH can also be regenerated using the secondary alcohol dehydrogenase *per se* of the present invention by adding the substrate for the oxidation reaction such as isopropanol to the reaction system.

### Brief Description of Drawings

Fig. 1 shows sodium dodecyl sulfate-polyacrylamide gel electrophoretic patterns of purified secondary alcohol dehydrogenase. Lane 1 stands for the purified secondary alcohol dehydrogenase and lane 2 for molecular weight markers.

Fig. 2 shows the process of purification of the secondary alcohol dehydrogenase.

Fig. 3 shows pH dependency of the secondary alcohol dehydrogenase in the oxidation reaction of (S)-1,3-butanediol.

Fig. 4 shows pH dependency of the secondary alcohol dehydrogenase in the reduction reaction of 4-hydroxy-2-butanone.

Fig. 5 shows temperature dependency of the secondary alcohol dehydrogenase in the oxidation reaction of (S)-1,3-butanediol.

Fig. 6 shows residual activity of the secondary alcohol dehydrogenase after treatment at 30°C for 30 minutes at different pHs.

Fig. 7 shows residual activity of the secondary alcohol dehydrogenase after treatment for 10 minutes at different temperatures.

### Best Mode for Carrying out the Invention

The present invention will be further demonstrated in detail below with reference to the following Examples, which are not construed to limit the scope of the present invention.

### Example 1

### Purification of secondary alcohol dehydrogenase

Cells of *Geotrichum candidum* IFO 4601 were cultured in YM medium (10 g of glucose, 5 g Bacto-peptone, 3 g yeast extract, 3g/L wheat germ extract, pH 6.0) and recovered by centrifugation. The wet cells thus obtained were disrupted using an ultrahigh pressure cell disrupter, and cell debris were removed by centrifugation to obtain cell free extract. Protamine sulfate was added to the resulting extract and nucleic acids and microsomes were removed. Centrifugation was performed to obtain the supernatant to which ammonium sulfate was added and the fractions precipitated with 30-70% saturated ammonium sulfate were recovered. Anion exchange chromatography was then carried out using DEAE-Toyopearl followed by density-gradient elution with sodium chloride to recover the peak fractions showing secondary alcohol dehydrogenase activity. The fractions thus obtained were applied to Blue-Sepharose affinity column and the effluent fractions passed through the column were recovered. The resulting fractions were subjected to hydrophobic chromatography using Butyl-Toyopearl followed by gradient elution using 33-0% saturated ammonium sulfate. The peak fractions showing secondary alcohol dehydrogenase activity were recovered. The fractions were then purified by HPLC gel filtration using TSK Gel G3000SW. The active fractions were further purified by anion exchange chromatography using Mono Q.

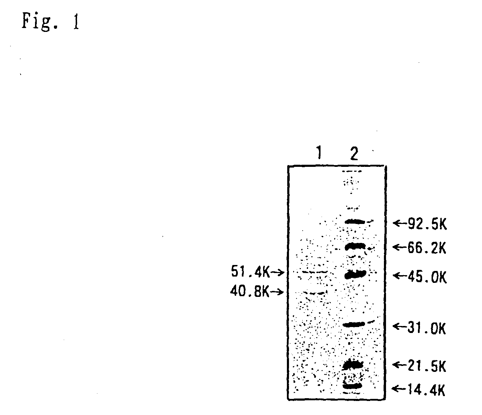

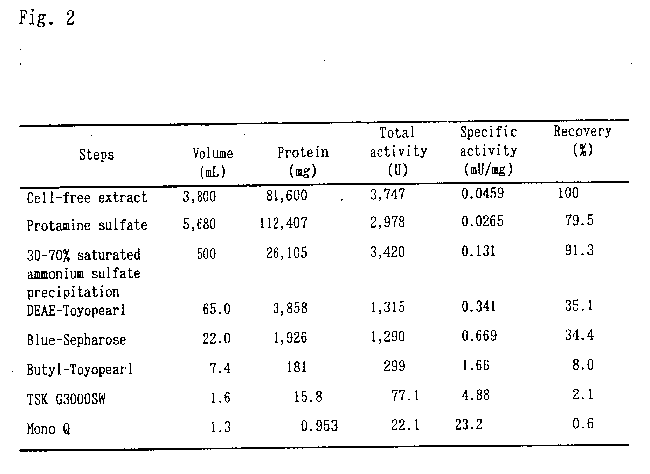

The thus-obtained secondary alcohol dehydrogenase preparation showed two protein bands when it was subjected to polyacrylamide gel electrophoresis. As a result of active staining, the protein with the lower mobility was found to be the secondary alcohol dehydrogenase. The molecular weight of this band was 51,000 (Fig. 1). The molecular weight of the purified enzyme determined by using gel filtration analysis column of TSK Gel G3000SW was found to be 107,000. Fig. 2 shows the purification scheme. Specific activity of the purified enzyme was 22.1 U/mg protein.

### Example 2

### Optimum pH for the secondary alcohol dehydrogenase

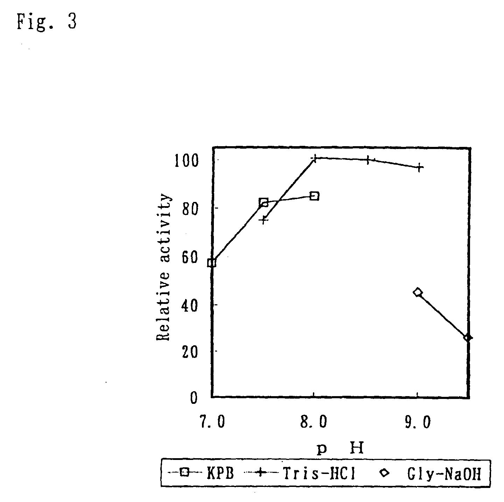

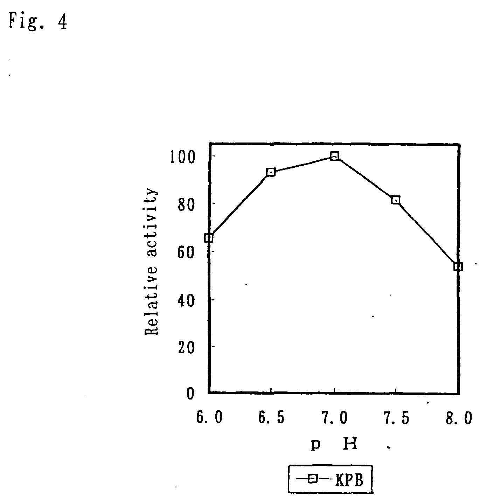

The secondary alcohol dehydrogenase was examined for its activity of oxidation of (S)-1,3-butanediol and that of reduction of 4-hydroxy-2-butanone [ which was measured under the same conditions as those used for measuring activity of oxidation of (S)-1,3-butanediol except for using NADH (0.4 µmol) instead of NAD⁺] at different pHs using potassium-phosphate buffer (KPB), Tris-HCl buffer and Britten-Robinson buffer by measuring the decrease of absorbance at 340 nm accompanied with reduction of the amount of NADH. The activity is shown in Figs. 3 and 4 as relative activity with taking the maximal activity as 100. The optimum pH in the reaction was 8.0-9.0 in the case of the oxidation of (S)-1,3-butanediol (Fig. 3) and 7.0 in the case of the reduction of 4-hydroxy-2-butanone (Fig. 4).

### Example 3

### Optimum temperature of the secondary alcohol dehydrogenase

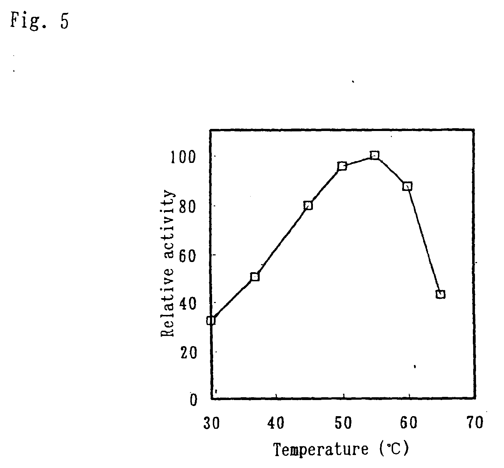

Activity of the secondary alcohol dehydrogenase was measured under the standard reaction conditions except for varying only temperature. The activity is shown in Fig. 5 as relative activity to the maximal activity that is taken as 100. The optimum temperature for oxidation of (S)-1,3-butanediol was 55°C.

### Example 4

### pH stability of the secondary alcohol dehydrogenase

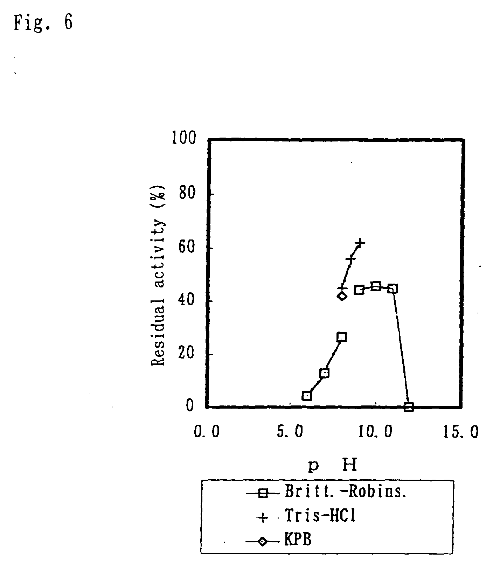

The enzyme was treated at 30°C for 30 minutes in Tris-HCl buffer, pH 8.0-9.0 or Britton-Robinson buffer, pH 5.0-12.0 and its residual activity was measured. The activity is shown in Fig. 6 as relative activity to the maximal activity that is taken as 100. The enzyme was the stablest at the pH ranging from 9.0 to 11.0.

### Example 5

### Thermostability of the secondary alcohol dehydrogenase

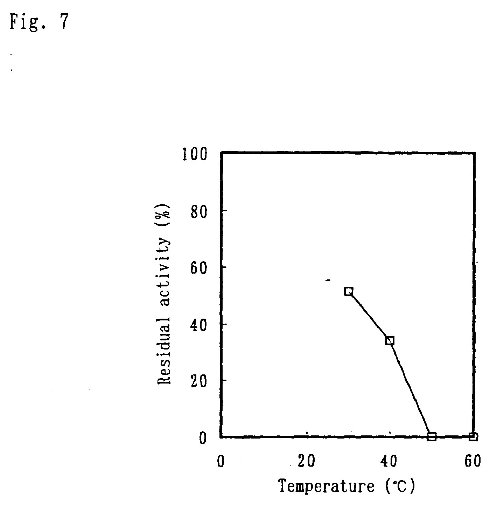

The secondary alcohol dehydrogenase was kept at pH 8.0 for 10 minutes and its residual activity was measured. It is shown in Fig. 7 as relative activity to the maximal activity that is taken as 100. The residual activity at 30°C was about 51%.

### Example 6

### Substrate specificity of the secondary alcohol dehydrogenase

The secondary alcohol dehydrogenase was reacted with various alcohols and aldehydes. Its oxidation and reduction activities are shown in Table 1 as relative activities to (S)-1,3-butanediol-oxidizing activity and 4-hydroxy-2-butanone-reducing activity that are taken as 100.

**Table 1**

| Substrates | Substrate concentration (mM) | Coenzyme | Relative activity (%) |
|---|---|---|---|
| Oxidation reaction | | | |
| (S)-1,3-butanediol | 50 | NAD⁺ | 100 |
| | 50 | NADP⁺ | 2.4 |
| (R)-1,3-butanediol | 50 | NADP⁺ | 8.8 |
| (S)-1-phenylethanol | 50 | NAD⁺ | 267 |
| (R)-1-phenylethanol | 50 | NAD⁺ | 19.2 |
| (S)-2-octanol | 5 | NAD⁺ | 187 |
| (R)-2-octanol | 5 | NAD⁺ | 13.3 |
| methyl (S)-3-hydroxybutyrate | 50 | NAD⁺ | 138 |
| methyl (R)-3-hydroxybutyrate | 50 | NAD⁺ | 22.1 |
| (RS)-2-butanol | 100 | NAD⁺ | 85.9 |
| (S)-2-butanol | 50 | NAD⁺ | 112 |
| (R)-2-butanol | 50 | NAD⁺ | 60.0 |
| (S)-1,2-propanediol | 50 | NAD⁺ | 23.8 |
| (R)1,2-propanediol | 50 | NAD⁺ | 10.4 |
| 2-propanol | 100 | NAD⁺ | 96.8 |
| cyclohexanol | 20 | NAD⁺ | 90.4 |
| methanol | 100 | NAD⁺ | 60.8 |
| ethanol | 100 | NAD⁺ | 10.7 |
| allyl alcohol | 100 | NAD⁺ | 18.7 |
| 1-propanol | 100 | NAD⁺ | 15.5 |
| 1-butanol | 100 | NAD⁺ | 12.9 |
| glycerol | 50 | NAD⁺ | 0 |

| Reduction reaction | | | |
|---|---|---|---|
| 4-hydroxy-2-butanone | 20 | NADH | 100 |
| | 20 | NADPH | 0 |
| acetone | 100 | NADH | 164 |
| acetophenone | 20 | NADH | 122 |
| ethyl acetoacetate | 100 | NADH | 96.8 |
| 2-butanone | 100 | NADH | 20.2 |

### Example 7

### Behavior of the secondary alcohol dehydrogenase to inhibitors

The secondary alcohol dehydrogenase was treated with various reagents at 30°C for 10 minutes. The resulting residual activities are shown in Table 2.

**Table 2**

| Inhibitors | Concentration (mM) | Residual activity (%) |
|---|---|---|
| ethylenediaminetetraacetic acid | 1.0 | 63.5 |
| | 10 | 9.8 |
| zinc chloride | 10 | 0.9 |
| cobalt chloride | 1.0 | 81.0 |
| copper sulfate | 1.0 | 71.5 |
| p-chloromercuribenzoic acid | 0.05 | 0.0 |
| iodoacetamide | 1.0 | 2.4 |
| dithiothreitol | 1.0 | 42.9 |
| 2-mercaptoethanol | 0.01 | 6.8 |
| mercuric chloride | 1.0 | 0.0 |

The enzyme was markedly inhibited by iodoacetamide, parachloromercuribenzoic acid, mercuric chloride, zinc chloride, concentrated metal chelator, and 2-mercaptoethanol.

### Example 8

### Enzyme kinetic analysis

The reaction rate constants of the purified secondary alcohol dehydrogenase to the following substrates were measured. As a result, the Km value to (S)-1,3-butanediol was 41.4 mM with Vmax of 36.7 U/mg of protein, while the Km value to (R)-1,3-butanediol was 165 mM with Vmax of 4.43 U/mg of protein and E value of 33.0.

### Industrial Applicability

Secondary alcohol dehydrogenase having broad specificity to substrates and high stereoselectivity is provided. The use of this enzyme provides a method of efficiently producing alcohols and ketones, particularly optically active alcohols and ketones.

## Claims

1. An enzyme having the following physicochemical properties (1) to (3):
(1) Action
It generates ketone or aldehyde by oxidizing alcohol in the presence of NAD⁺ (nicotinamide adenine dinucleotide) as a coenzyme and also generates alcohol by reducing ketone or aldehyde in the presence of NADH (reduced form of nicotinamide adenine dinucleotide) as a coenzyme;
(2) Substrate specificity
Substrates of the enzyme in the oxidation reaction are aliphatic alcohols that may be substituted with an aromatic group. Activity of the enzyme on secondary alcohols is higher than that on primary alcohols. S-forms of phenylethanol are preferentially oxidized. Substrates of the enzyme in the reduction reaction are aliphatic aldehydes or ketones that may be substituted with an aromatic group; and
(3) Molecular weight
Molecular weight of the enzyme is approximately 51,000, when determined by SDS-PAGE, while it is approximately 107,000, when determined by gel filtration.

2. A method of producing the enzyme according to claim 1, which comprises culturing a microorganism belonging to genus *Geotrichum* and producing the enzyme of claim 1 and recovering said enzyme from the culture.

3. The method according to claim 2, wherein said microorganism belonging to genus *Geotrichum* is *Geotrichum candidum.*

4. A DNA coding for the enzyme of claim 1.

5. A vector containing the DNA of claim 4.

6. A transformant carrying the vector of claim 5.

7. A method of producing alcohol, which comprises allowing the enzyme of claim 1 or transformant producing said enzyme, or its treated product to act on ketone or aldehyde to reduce said ketone or aldehyde.

8. A method of producing optically active alcohol, which comprises allowing the enzyme of claim 1 or transformant producing said enzyme, or its treated product to act on asymmetric ketone to reduce said asymmetric ketone.

9. A method of producing ketone or aldehyde, which comprises allowing the enzyme of claim 1 or transformant producing said enzyme, or its treated product to act on alcohol to oxidize said alcohol.

10. A method of producing optically active alcohol, which comprises allowing the enzyme of claim 1 or transformant producing said enzyme, or its treated product to act on racemic alcohol, preferentially oxidizing one form of optically active alcohol, and recovering the other form of the optically active alcohol.
